# EUROPÄISCHE PATENTSCHRIFT

(11) **EP 0 977 729 B1**
(45) Veröffentlichungstag und Bekanntmachung des Hinweises auf die Patenterteilung: **13.03.2002**
(21) Anmeldenummer: 98919266.1
(22) Anmeldetag: 14.04.1998
(51) Int. Cl.: C07C 255/57, C07C 251/48, C07C 65/30, C07C 25/13

(54) **3-CYANO-2,4,5-TRIFLUOR-BENZOYLFLUORID UND ZWISCHENPRODUKTE ZU SEINER HERSTELLUNG**
3-CYANO-2,4,5-TRIFLUORO-BENZOYL FLUORIDE AND INTERMEDIATE PRODUCTS FOR THE PRODUCTION THEREOF
FLUORURE DE 3-CYANO-2,4,5-TRIFLUORO-BENZOYLE ET PRODUITS INTERMEDIAIRES POUR SA FABRICATION

(30) Priorität: 24.04.1997 DE 19717231
(43) Veröffentlichungstag der Anmeldung: 09.02.2000
(73) Patentinhaber: BAYER AG, 51368 Leverkusen (DE)
(72) Erfinder: MARHOLD, Albrecht, D-51373 Leverkusen (DE); WOLFRUM, Peter, D-40789 Monheim (DE)
(86) Internationale Anmeldenummer: EP9802175
(87) Internationale Veröffentlichungsnummer: WO9847862

(56) Entgegenhaltungen:
- EP-A- 0 307 897
- WO-A-97/31001
- DE-A- 3 615 767
- DE-A- 3 702 393

## Beschreibung

Die vorliegende Erfindung betrifft 3-Cyano-2,4,5-trifluor-benzoylfluorid, Verfahren zu seiner Herstellung sowie weitere neue Halogenbenzol-Derivate als Zwischenprodukte.

3-Cyano-2,4,5-trifluor-benzoylchlorid kann zur Herstellung von antünfektiven Chinoloncarbonsäuren verwendet werden (vgl. DE-A 1 963 805 Nr.19 606 762.6 = WO 97/31001). Die Herstellung geht von der 2,4-Dichlor-5-fluor-3-cyanobenzoesäure aus und führt auf bekanntem Wege (vgl. DE 3702393 A1) zum 2,4-Dichlor-3-cyano-5-fluor-benzoylchlorid, welches dann fluoriert wird. Nachteilig an diesem Verfahren ist insbesondere die mit schlecht reproduzierbarer Ausbeute verlaufende Sandmeyer-Reaktion mit molarer Menge Kupfercyanid und einem zusätzlichen dreifachen Überschuß Natriumcyanid zur 2,4-Dichlor-3-cyano-5-fluor-benzoesäure. Der Einsatz solch großer Mengen Cyanid birgt bei der technische Durchführung auch ein erhebliches Gefahrenpotential.

Die vorliegende Erfindung betrifft das neue 3-Cyano-2,4,5-trifluor-benzoylfluorid der Formel (I)

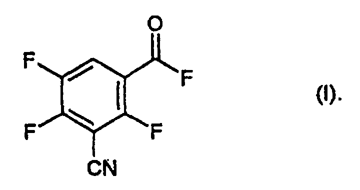

Die Erfindung betrifft auch ein Verfahren zur Herstellung von 3-Cyano-2,4,5-trifluorbenzoylchlorid durch Chlorieren von 3-Cyano-2,4,5-trifluor-benzoylfluorid.

Die Erfindung betrifft weiterhin auch die Verwendung von 3-Cyano-2,4,5-trifluorbenzoylfluorid zur Synthese von Chinolonen.

Die Erfindung betrifft weiterhin ein mehrstufiges Verfahren zur Herstellung von 3-Cyano-2,4,5-trifluor-benzoylfluorid, das von 5-Fluor-1,3-xylol (VIII) ausgeht, dadurch gekennzeichnet, daß man 5-Fluor-1,3-xylol (VIII) zweifach in Gegenwart eines Katalysators unter ionischen Bedingungen im Kern zum 2,4-Dichlor-5-fluor-1,3-dimethylbenzol (VII) chloriert, dieses anschließend unter radikalischen Bedingungen in den Seitenketten zu 2,4-Dichlor-5-fluor-3-dichlonnethyl-1-trichlormethylbenzol (VI) chloriert, dieses über die gegebenenfalls zu isolierende 2,4-Dichlor-5-fluor-3-dichlormethylbenzoesäure (V) zur 2,4-Dichlor-5-fluor-3-formyl-benzoesäure (IV) verseift, deren Aldehydgruppe zur 2,4-Dichlor-5-fluor-3-*N*-hydroxyiminomethyl-benzoesäure (III) umsetzt, aus dieser, bei gleichzeitiger Umwandlung der Carboxylgruppe in die Chlorcarbonylgruppe, Wasser unter Verwendung eines Säurechlorides zum Nitril 2,4-Dichlor-3-cyano-5-fluor-benzoylchlorid (II) eliminiert und dieses abschließend einem Fluor/Chlor-Austausch unterwirft.

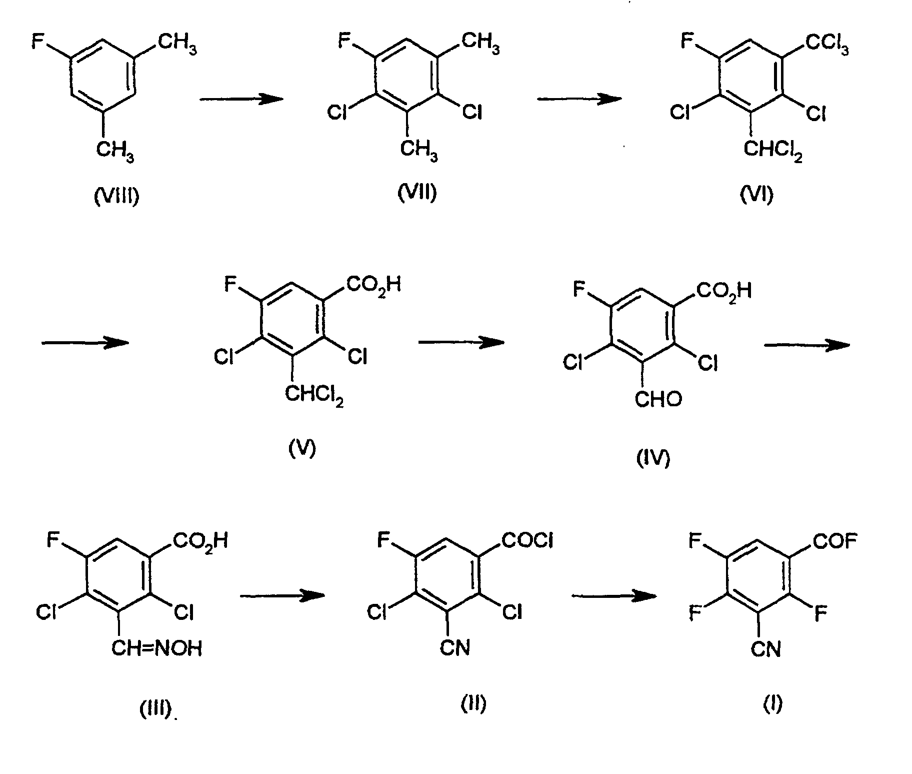

Alternativ kann die 2,4-Dichlor-5-fluor-3-formyl-benzoesäure (IV) auch zur 2,4-Dichlor-5-fluor-3-cyano-benzoesäure (IX) umgesetzt werden, die dann in das Säurechlorid (II) überführt werden kann.

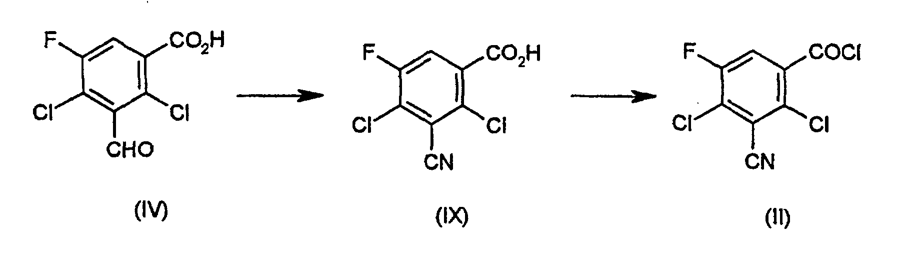

Die Zwischenprodukte der Formeln (III) bis (VII) sind ausnahmslos neu und ebenfalls Gegenstand der Erfindung.

3-Cyano-2,4,5-trifluor-benzoylfluorid ist ein durch das hier beschriebene Verfahren gut zugängliches Vorprodukt zur Herstellung von 3-Cyano-2,4,5-trifluor-benzoylchlorid.

Das erfindungsgemäße Verfahren wird im nachfolgenden näher ausgeführt.

Die Kernchlorierung von kommerziell erhältlichem 5-Fluor-1,3-xylol (VIII) zum 2,4-Dichlor-5-fluor-1,3-dimethylbenzol (VII) wird mit Chlorgas durchgeführt. 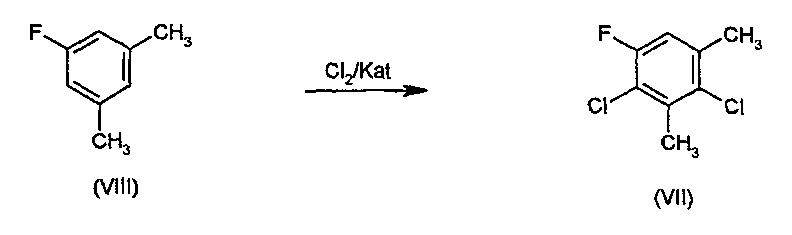 2,4-Dichlor-5-fluor-1,3-dimethylbenzol (VII) ist neu.

Als Katalysator setzt man einen oder mehrere Friedel-Crafts-Katalysatoren, vorzugsweise eine Lewis-Säure wie beispielsweise Eisen(III)-chlorid oder Aluminiumchlorid ein. Man setzt z.B. 0,1 bis 10 mol-%, vorzugsweise 0,2 bis 2 mol-% bezogen auf 5-Fluor-1,3-xylol ein.

Die Reaktion kann bei Temperaturen unterhalb von Raumtemperatur als auch leicht erhöhter Temperatur durchgeführt werden. Vorzugsweise arbeitet man zwischen 0 und 40°C.

Die Chlorierung kann in Substanz oder in einem geeigneten inerten Verdünnungsmittel durchgeführt werden. Als solches eignen sich besonders halogenierte Kohlenwasserstoffe wie Dichlor-, Trichlor-, Tetrachlormethan, 1,2-Dichlorethan oder 1,2,4-Trichlorbenzol. Die Chlorierung kann in kontinuierlicher oder diskontinuierlicher Fahrweise durchgeführt werden. Bei einem Konti-Prozeß ist es sinnvoll, nur bis zu einem niedrigen Umsatz zu fahren, weil die Chlorierung nicht mit vollständiger Selektivität erfolgt. Bei diskontinuierlicher Fahrweise leitet man Chlor bis zur ungefähr 0,8-bis 1,1-fachen, vorzugsweise 0,8- bis 0,95-fachen theoretischen Menge ein, wobei die Reaktionsmischung bei der Chlorierung ohne Verdünnungsmittel eine feste Konsistenz erreicht. Es ist auch hier vorteilhaft, nur bis zu diesem nicht zu hohen Umsatz zu fahren, weil dann die Verluste durch Überchlorierung auf der einen Seite und die Raum-Zeit-Ausbeute auf der anderen in einem optimalen Bereich liegen.

Die Aufarbeitung kann z.B. durch fraktionierte Destillation erfolgen. Es ist vorteilhaft, zurückgewonnenes Ausgangsmaterial und Monochlorverbindungen in den Prozeß zurückzuführen.

Die Seitenkettenchlorierung von 2,4-Dichlor-5-fluor-1,3-dimethylbenzol (VII) zu 2,4-Dichlor-5-fluor-3-dichlormethyl-1-trichlormethylbenzol (VI) führt man vorzugsweise in Substanz mit Chlorgas durch. 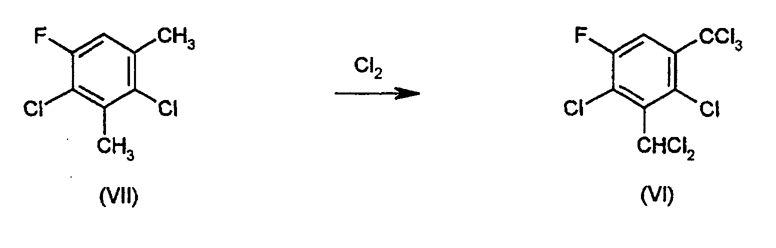 2,4-Dichlor-5-fluor-3-dichlormethyl-1-trichlormethylbenzol (VI) ist neu.

Die Bedingungen für die Radikalreaktion erreicht man durch erhöhte Temperatur und gegebenenfalls Bestrahlung mit einer Lichtquelle oder Zusatz eines üblichen Radikalstarters. Als Lichtquelle eignen sich Glühlampen wie vorzugsweise Halogenlampen oder Mittel- oder Hochdruck-Quecksilberdampflampen. Als Radikalstarter kommen z.B. Benzoylperoxid, Di-tert.-butylperoxid oder 2,2-Azo-bis-(isobuttersäurenitril) (AIBN) in Frage. Die Reaktionstemperatur kann zwischen 80 und 200°C, vorzugsweise 100 und 180°C, besonders bevorzugt zwischen 120 und 170°C liegen.

Die Chlorierung kann in kontinuierlicher oder diskontinuierlicher Fahrweise durchgeführt werden. Bei einem Konti-Prozeß ist es sinnvoll, nur bis zu einem niedrigen Umsatz zu fahren, weil die Chlorierung nicht mit vollständiger Selektivität erfolgt. Bei diskontinuierlicher Fahrweise leitet man Chlor ein bis zur ungefähr 0,8- bis 1,2-fachen, vorzugsweise 0,95- bis 1,15-fachen theoretischen Menge, entsprechend 40 bis 75 %, bevorzugt 65 bis 75 % Umsatz zum gewünschten Produkt.

Die Aufarbeitung kann z.B. durch fraktionierte Destillation oder Umkristallisieren aus einem geeigneten Lösungsmittel wie beispielsweise Methanol erfolgen. Bevorzugt wird die Destillation. Unterchlorierte Verbindungen können erneut in die Chlorierung eingesetzt werden.

Die Verseifung der chlorierten Seitenketten erfolgt mit einer Protonensäure gegebenenfalls in Gegenwart von Wasser. Hierzu eignen sich Mineralsäuren wie beispielsweise Schwefelsäure, Salzsäure oder Phosphorsäure und organische Säuren wie beispielsweise Ameisensäure, Essigsäure oder Oxalsäure sowie Mischungen daraus und mit einem protischen Lösungsmittel wie beispielsweise Wasser.

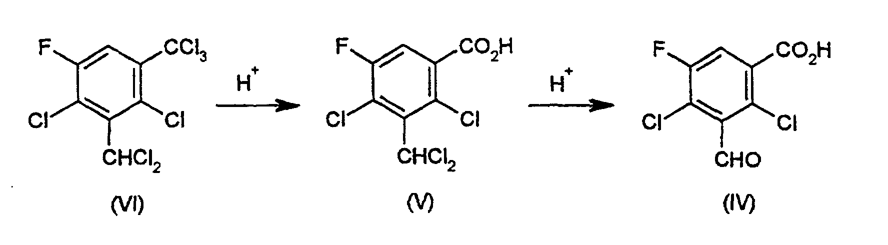

Je nach Art, Konzentration und Menge der Säure und Reaktionstemperatur kann man die Verseifung von 2,4-Dichlor-5-fluor-3-dichlormethyl-1-trichlormethylbenzol (VI) zur 2,4-Dichlor-5-fluor-3-formyl-benzoesäure (IV) in einem oder zwei Schritten durchführen. Weil die Trichlormethylgruppe wesentlich schneller verseift wird, kann man die 2,4-Dichlor-5-fluor-3-dichlormethylbenzoesäure (V) bei geeigneten Reaktionsbedingungen ohne weiteres isolieren und diese in einem weiteren Verseifungsschritt zu (IV) umsetzen. Das erfindungsgemäße Gesamtverfahren zur Herstellung von 3-Cyano-2,4,5-trifluor-benzoylfluorid betreffend ist es vorteilhaft, die Verseifung in einem Schritt durchzuführen.

2,4-Dichlor-5-fluor-3-formyl-benzoesäure (IV) und 2,4-Dichlor-5-fluor-3-dichlormethylbenzoesäure (V) sind neu.

Die Menge der Protonensäure ist nicht kritisch. Man legt diese z.B. vor und dosiert den geschmolzenen Aromat (VI) bzw. (V) hinzu. Vorzugsweise setzt man soviel Säure(gemisch) ein, daß die Reaktionsmischung rührbar bleibt.

Die Temperatur kann bei der Verseifung in Abhängigkeit vom gewünschten Produkt, Säure und Reaktionszeit in einem weiten Bereich variiert werden. Im allgemeinen arbeitet man bei 0 bis 100°C.

Die Isolierung des Produkts kann z.B. durch Ausfällung mit Wasser und Abtrennen durch Filtration oder Extraktion erfolgen.

Die Herstellung des Oxims (III) aus der 2,4-Dichlor-5-fluor-3-formyl-benzoesäure (IV) erfolgt nach allgemein bekannter Methode. 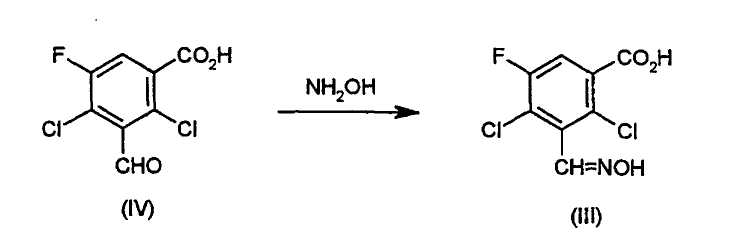 2,4-Dichlor-5-fluor-3-N-hydroxyiminomethyl-benzoesäure (III) ist neu.

Als Reagenz setzt man Salze des Hydroxylamin wie beispielsweise das Hydrochlorid oder Sulfat oder auch die freie Base ein.

Bei Einsatz von einem Salz des Hydroxylamins wird die Reaktion in Gegenwart eines Säureakzeptors durchgeführt. Als solcher kommen übliche anorganische oder organische Basen in Frage. Hierzu gehören vorzugsweise Erdalkalimetall- oder Alkalimetallhydroxide, -alkoholate, -acetate, -carbonate oder -hydrogencarbonate, wie beispielsweise Natrium-, Kalium- oder Ammoniumhydroxid, Natrium-methylat, Natriumethylat, Kalium-tert.-butylat, Natrium-, Kalium-, Calcium- oder Ammoniumacetat, Natrium-, Kalium- oder Ammoniumcarbonat, Natriumhydrogen- oder Kaliumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktion wird in Gegenwart eines Verdünnungsmittels durchgeführt. Als solches kommen Wasser, organische Lösungsmittel und beliebige Mischungen davon in Betracht. Beispielhaft seien genannt: Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Nitrile, wie Acetonitril, Propionitril, n- oder i-Butyronitril; Alkohole, wie Methanol, Ethanol, n- oder i-Propanol, n-, iso-, sek- oder tert-Butanol, Ethandiol, Propan-1,2-diol, Ethoxyethanol, Methoxyethanol, Diethylenglykolmonomethylether, Diethylenglykolmonoethylether; Wasser.

Die Temperatur bei der Reaktion kann in einem größeren Bereich variiert werden. Sie wird im allgemeinen zwischen 10 und 100°C, bevorzugt zwischen 20 und 80°C gewählt.

Man setzt pro Mol Formylbenzoesäure (IV) 1 bis 1,5 Äquivalente Hydroxylamin(-Salz) ein und 200 bis 2000 ml, vorzugsweise 500 bis 1000 ml Verdünnungsmittel ein. Pro Äquivalent Hydroxylamin setzt man 1 bis 5, vorzugsweise 1,1 bis 3 Äquivalente Säureakzeptor ein.

Zur Aufarbeitung säuert man die Reaktionsmischung z.B. mit einer Mineralsäure an und extrahiert gegebenenfalls das Produkt mit einem geeigneten Lösungsmittel wie beispielsweise Methyl-tert.-butyl-ether oder filtriert den Feststoff ab.

Wird die Umsetzung von (IV) mit Hydroxylamin oder einem Salz des Hydroxylamins in Gegenwart von Ameisensäure durchgeführt, so erhält man als Reaktionsprodukt das Nitril (IX). Bevorzugt wird das Hydroxylamin-hydrochlorid verwendet.

Die Reaktion kann in Gegenwart eines Verdünnungsmittels durchgeführt werden. In Frage kommen Wasser, organische Lösungsmittel und beliebige Mischungen davon in Betracht. Bevorzugt wird die Reaktion in 85 %- bis 98 %iger wäßriger Ameisensäure durchgeführt.

Die Temperatur der Reaktion kann in einem größeren Bereich variiert werden. Sie wird im allgemeinen zwischen 10 und 120°C, bevorzugt zwischen 50 und 110°C gewählt.

Man setzt pro Mol (IV) 1 bis 1,5 Äquivalente Hydroxylamin-(Salz) ein und 100 bis 3 000 ml, vorzugsweise 500 bis 1 500 ml, wäßrige Ameisensäure.

Die Edukte können in unterschiedlicher Reihenfolge zusammengegeben werden. So können alle Edukte vorgelegt werden und dann gemeinsam auf die Reaktionstemperatur aufgeheizt werden. Es kann aber auch das Hydroxylamin-(Salz) in der wäßrigen Ameisensäure vorgelegt werden und das Edukt (IV) bei der Reaktionstemperatur eingetragen werden.

Alternativ kann man das Edukt (IV), welches auch schwefelsäurefeucht eingesetzt werden kann, in der wäßrigen Ameisensäure vorlegen und das Hydroxylamin-(Salz) oder eine Lösung des Hydroxylamin-(Salz) in Wasser oder wäßriger Ameisensäure bei der Reaktiönstemperatur zudosieren.

Zur Aufarbeitung verdünnt man weiter mit Wasser und filtriert (IX) als Feststoff ab.

Die Überführung der 3-Cyano-2,4-dichlor-5-fluorbenzoesäure (IX) in das Benzoylchlorid (II) erfolgt durch Einsatz eines Chlorierungsmittels als Reagenz.

Als Chlorierungsmittel eignen sich die weiter unten angegebenen Mittel unter den dort angegebenen Reaktionsbedingungen.

Die Herstellung von 2,4-Dichlor-3-cyano-5-fluor-benzoylchlorid (II) durch Wasserabspaltung aus der 2,4-Dichlor-5-fluor-3-*N*-hydroxyiminomethyl-benzoesäure (III) bei gleichzeitiger Überführung der Carbonsäurefunktion in das Carbonsäurechlorid erfolgt durch Einsatz eines Chlorierungsmittels als Reagenz.

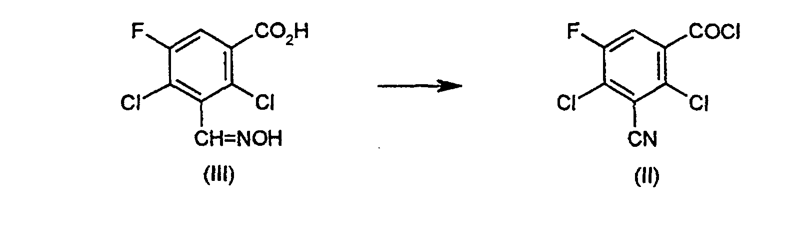

Als Chlorierungsmittel eignen sich anorganische oder organische Säurechloride wie beispielsweise Phosgen (Carbonyldichlorid) bzw. die synthetischen Äquivalente Chlorameisensäure-trichlormethylester oder Bis(trichlormethyl)-carbonat, Oxalylchlorid, Acetylchlorid, Thionylchlorid, Sulfurylchlorid, Phosphortrichlorid, Phosphorpentachlorid oder Phosphoroxychlorid und deren Gemische. Bevorzugt sind Phosgen oder Thionylchlorid.

Die Reaktion kann in Gegenwart oder Abwesenheit eines geeigneten Verdünnungsmittels durchgeführt werden. Hierfür kommen organische Lösungsmittel, ein unter den Reaktionsbedingungen flüssiges Säurechlorid, d.h. das Reagenz selber, und beliebige Mischungen davon in Betracht. Beispielhaft für organische Lösungsmittel seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol.

Die Zugabe von Oxim-Verbindung und Chlorierungsmittel kann in beliebiger Reihenfolge erfolgen. In einer bevorzugten Ausführungsform legt man das lösungsmittelfreie Reagenz vor und dosiert 2,4-Dichlor-5-fluor-3-*N*-hydroxyiminomethyl-benzoesäure im Maße einer kontrollierbaren Gasentwicklung (Chlorwasserstoff und gegebenenfalls weitere Gase wie Kohlendioxid oder Schwefeldioxid) kontinuierlich oder portionsweise hinzu.

Man setzt pro Mol Verbindung (III) im allgemeinen 2 bis 10 Mol Säurechlorid ein. Es ist auch möglich einen größeren Überschuß zu verwenden, insbesondere wenn das Säurechlorid auch als Verdünnungsmittel dient.

Die Temperatur bei der Reaktion kann in einem größeren Bereich variiert werden. Sie wird im allgemeinen zwischen 0 und 150°C, bevorzugt zwischen 30°C und Siedetemperatur gewählt. Dabei arbeitet man im allgemeinen unter Normaldruck. Es ist auch möglich unter verminderten oder erhöhtem Druck zu arbeiten. Z.B. ist es sinnvoll bei Einsatz von Phosgen dieses bei einer Temperatur oberhalb des Siedepunktes bei Normaldruck flüssig zu halten und die frei werdenden Gase über ein Druckhaltevorrichtung zu entspannen.

Die Aufarbeitung kann beispielsweise durch fraktionierte Destillation erfolgen.

Der abschließende Fluor/Chlor-Austausch erfolgt nucleophil mit einer Fluoridquelle.

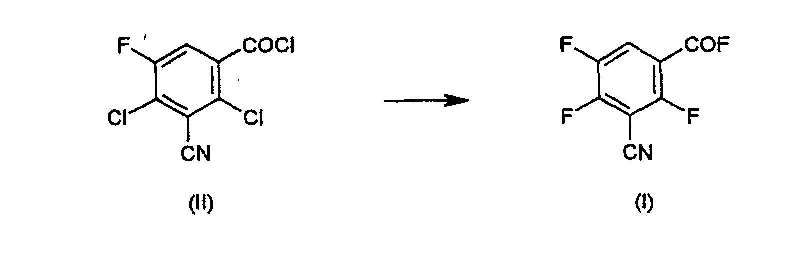

Geeignete Fluoridquellen sind beispielsweise Metallfluoride, bevorzugt Alkalimetallfluoride wie beispielsweise Kaliumfluorid oder Cäsiumfluorid.

Die Fluorierung wird in Gegenwart eines Verdünnungsmittels durchgeführt. Als solches geeignet sind polar aprotische Lösungsmittel wie beispielsweise Amide, wie N,N-Dimethylformamid, N,N-Dimethylacetamid, N-Methylformanilid, N-Methylpyrrolidon oder Hexamethylphosphorsäuretriamid; Harnstoffe, wie *N,N*-Dimethylpropylenharnstoff, *N,N*-Dimethyl-ethylenharnstoff; Sulfoxide, wie Dimethylsulfoxid; Sulfone, wie Sulfolan.

Die Fluorierung kann auch in Gegenwart bekannter Katalysatoren für Halex-Reaktionen durchgeführt werden.

Man setzt pro Mol 2,4-Dichlor-3-cyano-5-fluor-benzoylchlorid 3 bis 10 Mol, bevorzugt 3,4 bis 8 Mol, besonders bevorzugt 3,7 bis 6 Mol Fluorid ein.

Die Fluorierung wird bei erhöhter Temperatur durchgeführt. Man arbeitet im allgemeinen bei 100 bis 250°C, bevorzugt bei 130 bis 200°C.

Die Aufarbeitung kann beispielsweise erfolgen, indem man das Produkt unter vermindertem Druck abdestilliert oder mit einem Lösungsmittel extrahiert und anschließend fraktioniert destilliert.

Die erfindungsgemäße Herstellung von 3-Cyano-2,4,5-trifluor-benzoylchlorid aus 3-Cyano-2,4,5-trifluor-benzoylfiuorid (I) wird analog zu bekannten Verfahren zur Rückchlorierung von Carbonsäurefluoriden durchgeführt. Als Reagenzien eignen sich hierzu Siliciumchloride, wie beispielsweise Siliciumtetrachlorid, Trimethylchlorsilan oder Dimethyldichlorsilan; oder Calciumchlorid, jeweils in Gegenwart von katalytischen Mengen einer Lewis-Säure, wie beispielsweise Aluminiumchlorid oder Bortrichlorid; oder die genannten und andere chlorhaltige Lewissäuren selber.

Man setzt pro Mol Verbindung (I) im allgemeinen 1 bis 2 Äquivalente Reagenz und gegebenenfalls 0,01 bis 0,1 Mol Lewis-Säure ein.

Die Temperatur bei der Reaktion kann in einem größeren Bereich variiert werden. Im allgemeinen arbeitet man mit Siliciumchloriden und Lewis-Säuren bei 20 bis 150°C, mit Calciumchlorid bei 120 bis 200°C.

Die Aufarbeitung erfolgt vorzugsweise durch Vakuumdestillation.

Es ist ausgesprochen überraschend, daß die Kernchlorierung von 5-Fluor-1,3-xylol zum 2,4-Dichlor-5-fluor-1,3-dimethylbenzol mit sehr hoher Selektivität abläuft, ohne welche die erfindungsgemäße Synthesesequenz nicht möglich wäre.

Weitere Details zu den Verfahren gehen aus den folgenden Beispielen hervor ohne daß die Erfindung dadurch beschränkt wird.

### Beispiel 1

### Herstellung von 2,4-Dichlor-5-fluor-1,3-dimethylbenzol

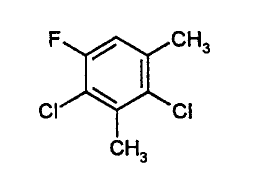

### a) lösungsmittelfrei

In 124 g 3,5-Dimethyl-fluorbenzol wurden 1 g wasserfreies Eisen(III)-chlorid vorgelegt und im Maße der Reaktion Chlor eingeleitet (ca. 4 h). Die Reaktion ist anfangs leicht exotherm (Temperaturanstieg von 24 auf 32°C) und wurde durch leichte Kühlung unter 30°C gehalten. Nach 120 g Chlor-Zufuhr wurde der Ansatz fest. Nach GC-Analyse waren 33,4 % Monochlorverbindung, 58,4 % gewünschtes Produkt und 5 % überchlorierte Verbindungen entstanden. Nach Abziehen des Chlorwasserstoffes wurde im Wasserstrahlvakuum an einer Kolonne destilliert.

Im Vorlauf wurden bei 72-74°C/22 mbar 49 g 2-Chlor-5-fluor-1,3-dimethylbenzol erhalten. Nach einem Zwischenlauf von 5 g gingen bei 105°C/22 mbar 75 g 2,4-Dichlor-5-fluor-1,3-dimethylbenzol über.
Schmelzbereich: 64 - 65°C

### b) in 1,2-Dichlorethan

1 kg 3,5-Dimethyl-fluorbenzol und 15 g wasserfreies Eisen(III)-chlorid wurden in 1 l 1,2-Dichlorethan vorgelegt und im Maße der Reaktion Chlor eingeleitet (ca. 4 h). Die Reaktion ist anfangs leicht exotherm (Temperaturanstieg von 24 auf 32°C) und wurde durch leichte Kühlung unter 30°C gehalten. Nach 1200 g Chloraufhahme waren laut GC-Analyse 4 % Monochlorverbindung, 81,1 % gewünschtes Produkt und 13,3 % überchlorierte Verbindungen entstanden. Nach Abdestillieren des Lösungsmittels bzw. Chlorwasserstoffes wurde im Wasserstrahlvakuum an einer Kolonne destilliert.

Im Vorlauf wurden 40 g 2-Chlor-5-fluor-1,3-dimethylbenzol erhalten. Nach wenig Zwischenlauf gingen bei 127 - 128°C/50 mbar 1115 g 2,4-Dichlor-5-fluor-1,3-dimethylbenzol über.

### Beispiel 2

### 2,4-Dichlor-5-fluor-3-dichlormethyl-1-trichlormethylbenzol

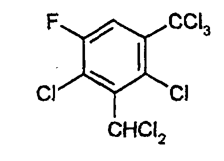

In einer Photochlorierungsapparatur mit Chloreinleitung und Ableitung für den Chlorwasserstoff zu einem Wäscher und einer Lichtquelle in der Nähe des Chloreinleitungsrohrs wurden 1890 g 2,4-Dichlor-5-fluor-1,3-dimethylbenzol vorgelegt und bei 140 bis 150°C Chlor eindosiert. Nach 30 h waren 3850 g Chlor eingeleitet. Der Gehalt an gewünschtem Produkt nach GC-Analyse betrug 71,1 %; der Anteil an minderchlorierten Verbindungen 27,7 %.

Destillaton über eine 60 cm-Kolonne mit Wilson-Spiralen lieferte einen Vorlauf von 1142 g, der erneut in die Chlorierung eingesetzt werden konnte. Der Hauptlauf bei 160-168°C/0,2 mbar lieferte 2200 g 2,4-Dichlor-5-fluor-3-dichlormethyl-1-trichlormethylbenzol mit einem Schmelzbereich von 74-76°C.

Nach Umkristallisieren einer Probe aus Methanol betrug der Schmelzpunkt 81-82°C. Der Vorlauf aus dieser Reaktion kann mit neuem Edukt (1 555 g) erneut in die Chlorierung eingesetzt werden. Dabei wurden 1 377 g Vorlauf und 2 465 g Hauptlauf mit einer Reinheit von 97,4 % erhalten.

### Beispiel 3

### 2,4-Dichlor-5-fluor-3-formyl-benzoesäure

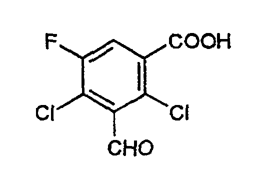

In einer Rührapparatur mit Gasableitung wurden 2500 ml 95%-ige Schwefelsäure bei 70°C vorgelegt und unter Rühren 500 g aufgeschmolzenes 2,4-Dichlor-5-fluor-3-dichlormethyl-1-trichlormethylbenzol zugetropft. Es setzte nach kurzer Zeit Chlorwasserstoffentwicklung ein. Nach 2 h war alles eindosiert und es wurde bis zum Ende der Gasentwicklung nachgerührt. Nach Abkühlen auf 20°C wurde der Ansatz auf 4 kg Eis ausgetragen und der ausgefallene Feststoff abgesaugt. Das Produkt wurde mit Wasser nachgewaschen und getrocknet. Die Ausbeute an 2,4-Dichlor-5-fluor-3-formyl-benzoesäure betrug 310 g.
Schmelzbereich: 172-174°C

Eine Wiederholung des Versuchs mit 2 390 g Edukt, 7 170 ml Schwefelsäure ergab 1 540 g Produkt (97,8 % Reinheit).

### Beispiel 4

### 2,4-Dichlor-5-fluor-3-N-hydroxyiminomethyl-benzoesäure

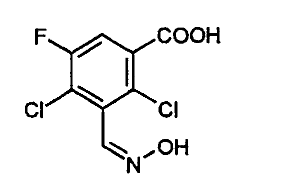

In einer Rührapparatur wurden 80 g Hydroxylammoniumchlorid in 500 ml Ethanol vorgelegt und 200 ml 45 %-ige Natronlauge zugetropft und anschließend bei 40-45°C 200 g 2,4-Dichlor-5-fluor-3-formyl-benzoesäure eingetragen. Die Reaktion war leicht exotherm und es wurde 5 h bei 60°C nachgerührt. Nach Abkühlen auf Raumtemperatur wurde durch Zutropfen von Salzsäure auf pH < 3 gestellt. Das Produkt wurde in tert.-Butyl-methyl-ether aufgenommen und die organische Phase abgetrennt. Nach Abdestillieren des Lösungsmittels verblieben 185 g 2,4-Dichlor-5-fluor-3-*N*-hydroxyiminomethyl-benzoesäure.
Schmelzbereich: 190-194°C

Eine Wiederholung dieses Beispiels in Wasser anstelle von Ethanol mit 60,5 g Hydroxylammoniumchlorid in 363 ml Wasser, 150 ml 45 % NaOH, 150g Edukt ergab 150 g Produkt (93,4 % Reinheit).

### Beispiel 5

### 2,4-Dichlor-3-cyano-5-fluor-benzoylchlorid

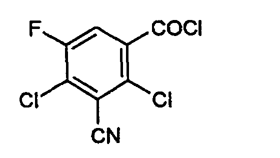

In einer Rührapparatur mit Dosiervorrichtung und Gasableitung über einen Rückflußkühler zu einem Wäscher wurden 600 ml Thionylchlorid vorgelegt und bei 20°C 210 g Ausgangsmaterial im Maße der Chlorwasserstoff-/Schwefeldioxid-Entwicklung eingetragen. Nach Ende der Zugabe wurde auf Rückfluß erhitzt bis die Gasentwicklung zu Ende ging. Danach wurde destilliert. Im Siedebereich von 142-145°C/10 mbar wurden 149 g 2,4-Dichlor-3-cyano-5-fluor-benzoylchlorid erhalten (Gehalt nach GC; 98,1 %).
Schmelzbereich: 73-75°C

Eine Wiederholung dieses Beispiels mit 450 g Phosphoroxychlorid in 200 ml Chlorbenzol als Chlorierungsmittel ergab aus 80 g Edukt (80 %ig) 48 g Produkt (89,0 % Reinheit).

### Beispiel 6

### 3-Cyano-2,4,5-trifluor-benzoylfluorid

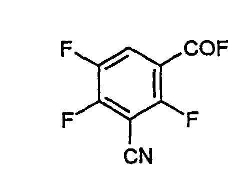

50 g Kaliumfluorid wurden in 120 ml Tetramethylensulfon suspendiert und bei 15 mbar zur Trocknung andestilliert (ca. 20 ml). Anschließend wurden 50,4 g 2,4-Dichlor-3-cyano-5-fluor-benzoylchlorid zugefügt und unter Feuchtigkeitsausschluß für 12 Stunden bei 180°C Innentemperatur gerührt. Durch Vakuumdestillation wurden 32,9 g 3-Cyano-2,4,5-trifluor-benzoylfluorid im Siedebereich von 98-100°C/12 mbar erhalten.

### Beispiel 7

### 3-Cyano-2,4,5-trifluor-benzoylchlorid

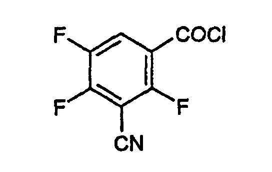

76,6 g 3-Cyano-2,4,5-trifluor-benzoylfluorid wurden zusammen mit 1 g wasserfreiem Aluminiumchlorid bei 60-65°C vorgelegt und dann im Zuge der Gasentwicklung 25 g Siliciumtetrachlorid zugetropft. Nach Ende der Gasentwicklung bei 65°C wurde im Vakuum destilliert. Im Siedebereich von 120-122°C/14 mbar gingen 73,2 g 3-Cyano-2,4,5-trifluor-benzoylchlorid über.

### Beispiel 8

### 3-Cyano-2.4-dichlor-5-fluorbenzoesäure

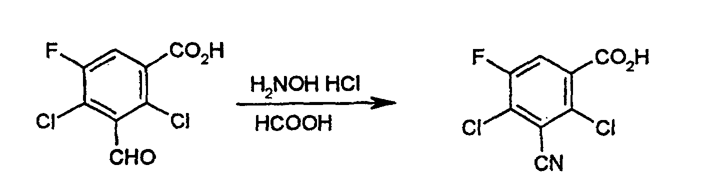

162 g Hydroxylaminhydrochlorid wurden in 2 000 ml Ameisensäure (technisch, 85 %ig) vorgelegt. Bei 95°C wurden 950 g 2,4-Dichlor-5-fluor-3-formylbenzoesäure (schwefelsäurefeucht, 42 %ig) eingetragen. Dabei trat kurzes Aufschäumen ein, dann erhielt man sofort eine klare Lösung. Das Gemisch wurde dann 4 Stunden bei 100 bis 105°C (Rückfluß) gerührt.

Nach dem Abkühlen auf Raumtemperatur wurde auf Wasser gegeben, sehr gut durchrührt, abgesaugt und getrocknet. Man erhielt so 364 g (90,3 % der Theorie) 3-Cyano-2,4-dichlor-5-fluorbenzoesäure mit einem Gehalt von GC von 97,7 %.

### Beispiel 9

### 3-Cyano-2,4-dichlor-5-fluorbenzoylchlorid

In einer Rührapparatur mit Dosiervorrichtung und Gasableitung über einen Rückflußkühler zu einem Wäscher wurden 4 100 ml Thionylchlorid und 41 ml Pyridin vorgelegt, und bei 20°C 2 050 g 3-Cyano-2,4-dichlor-5-fluorbenzoesäure (99,5 %ig) im Maße der Chlorwasserstoff-/Schwefeldioxid-Entwicklung eingetragen. Nach Ende der Zugabe wurde auf Rückfluß erhitzt bis die Gasentwicklung zu Ende ging. Danach wurde destilliert. Im Siedebereich von 142 bis 145°C/10 mbar wurden 2 150 g (95,7 % der Theorie) 3-Cyano-2,4-dichlor-5-fluorbenzoylchlorid erhalten (Gehalt nach GC: 98,0 %).

## Patentansprüche

1. 3-Cyano-2,4,5-trifluor-benzoylfluorid der Formel (I)

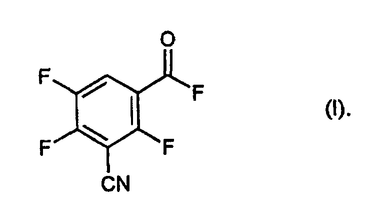

2. Verfahren zur Herstellung von 3-Cyano-2,4,5-trifluor-benzoylfluorid der Formel (I) gemäß Anspruch 1, **dadurch gekennzeichnet, daß** man 3-Cyano-2,4-dichlor-5-fluor-benzoylchlorid nucleophil mit einem Fluoridierungsmittel umsetzt gemäß folgendem Schema:

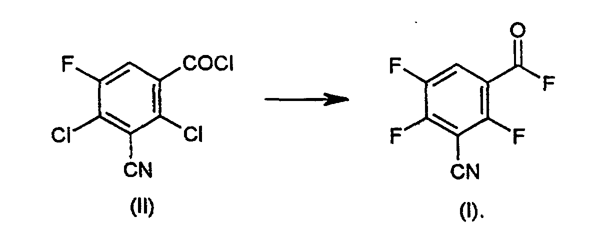

3. Verwendung von 3-Cyano-2,4,5-trifluoro-benzoylfluorid zur Herstellung von 3-Cyano-2,4,5-trifluor-benzoylchlorid durch Umsetzung von 3-Cyano-2,4,5-trifluor-benzoylfluorid mit Chlorierungsmitteln, gegebenenfalls in Gegenwart von Verdünnungsmitteln.

4. Verfahren zur Herstellung von 2,4-Dichlor-3-cyano-5-fluor-benzoylchlorid der Formel (II) durch Wasserabspaltung aus der 2,4-Dichlor-5-fluor-3-N-hydroxyiminomethyl-benzoesäure (III) bei gleichzeitiger Überführung der Carbonsäurefunktion in das Carbonsäurechlorid nach folgendem Schema:

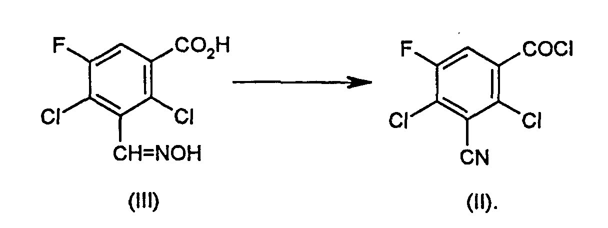

5. 2,4-Dichlor-5-fluor-3-N-hydroxyimino-methyl-benzoesäure der Formel (III)

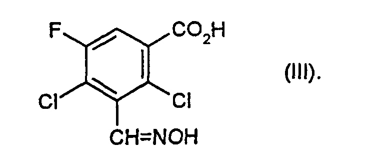

6. Verfahren zur Herstellung von 2,4-Dichlor-5-fluor-3-N-hydroxyiminomethylbenzoesäure der Formel (III) gemäß Anspruch 5, durch Umsetzung von 2,4-Dichlor-5-fluor-3-formyl-benzoesäure der Formel (IV) mit Hydroxylamin nach folgendem Schema:

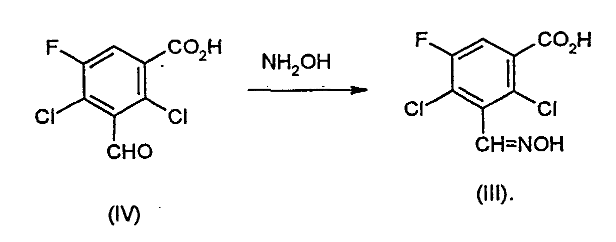

7. Verfahren zur Herstellung von 2,4-Dichlor-3-cyano-5-fluor-benzoesäure (IX)
a) durch Wasserabspaltung aus 2,4-Dichlor-5-fluor-3-N-hydroxyiminomethyl-benzoesäure der Formel (III) nach folgendem Schema 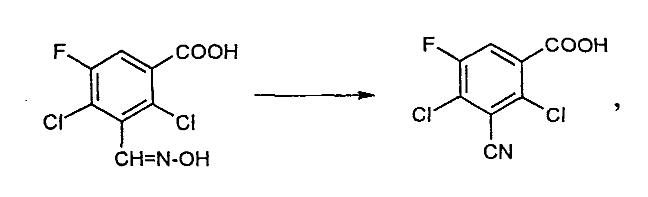
b) durch Umsetzung von 2,4-Dichlor-5-fluor-3-formyl-benzoesäure der Formel (IV) mit Hydroxylamin in Gegenwart von Ameisensäure nach folgendem Schema

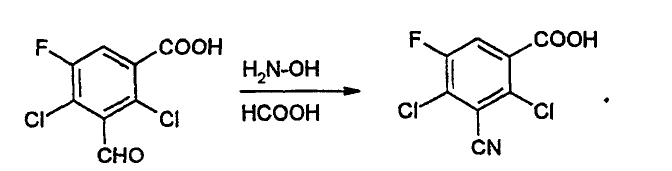

8. 2,4-Dichlor-5-fluor-3-formyl-benzoesäure der Formel (IV)

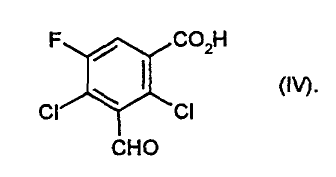

9. Verfahren zur Herstellung von 2,4-Dichlor-5-fluor-3-formyl-benzoesäure der Formel (IV) gemäß Anspruch 7, 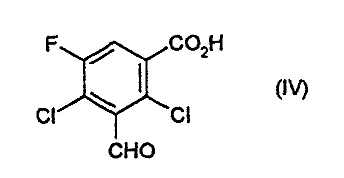
a) durch Verseifung von 2,4-Dichlor-5-fluor-3-dichlormethyl-benzoesäure oder
b) durch Verseifung von 2,4-Dichlor-5-fluor-3-dichlormethyl-1-trichlormethylbenzol der Formel (VI),
jeweils in Gegenwart von Säuren und gegebenenfalls in protischen Lösungsmitteln.

10. 2,4-Dichlor-5-fluor-3-dichlormethyl-1-trichlormethylbenzol der Formel (VI)

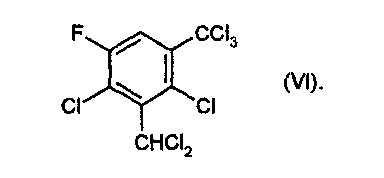

11. Verfahren zur Herstellung von 2,4-Dichlor-5-fluor-3-dichlormethyl-1-trichlormethylbenzol der Formel (VI), 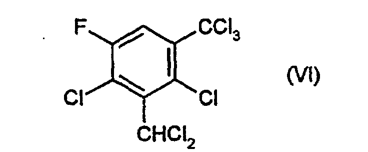 gemäß Anspruch 9 durch Seitenkettenchlorierung von 2,4-Dichlor-5-fluor-1,3-dimethylbenzol (VII) zu 2,4-Dichlor-5-fluor-3-dichlormethyl-1-trichlormethylbenzol (VI) gemäß folgendem Schema: 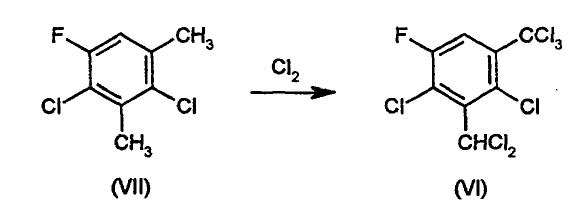 **dadurch gekennzeichnet, daß** man die Chlorierung unter radikalischen Bedingungen und/oder bei erhöhter Temperatur durchführt.

12. 2,4-Dichlor-5-fluor-1,3-dimethylbenzol der Formel (VII)

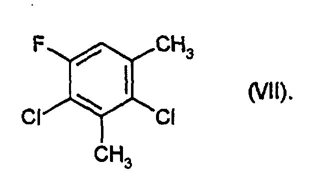

13. Verfahren zur Herstellung 2,4-Dichlor-5-fluor-1,3-dimethylbenzol der Formel (VII) gemäß Anspruch 11 durch Kernschlorierung von 5-Fluor-1,3-xylol (VIII) zum 2,4-Dichlor-5-fluor-1,3-dimethylbenzol (VII) mit Chlorgas, gegebenenfalls in einem Verdünnungsmittel und in Gegenwart eines Katalysators gemäß folgendem Schema:

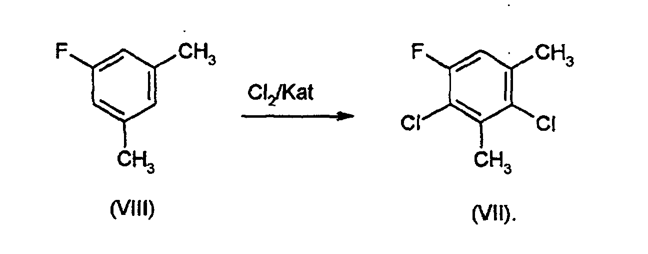

## Claims

1. 3-Cyano-2,4,5-trifluoro-benzoyl fluoride of the formula (I)

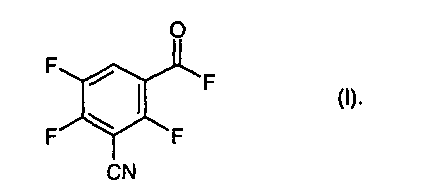

2. Process for the preparation of 3-cyano-2,4,5-trifluoro-benzoyl fluoride of the formula (I) according to Claim 1, **characterized in that** 3-cyano-2,4-dichloro-5-fluoro-benzoyl chloride is reacted nucleophilically with a fluoridizing agent according to the following equation:

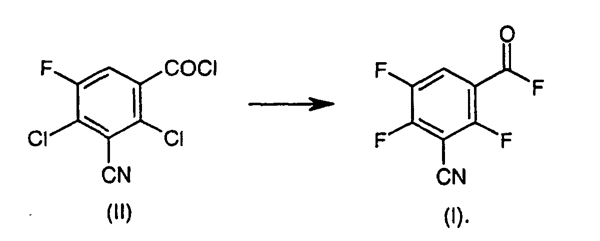

3. Use of 3-cyano-2,4,5-trifluoro-benzoyl fluoride for the preparation of 3-cyano-2,4,5-trifluorobenzoyl chloride by reaction of 3-cyano-2,4,5-trifluoro-benzoyl fluoride with chlorinating agents, optionally in the presence of diluents.

4. Process for the preparation of 2,4-dichloro-3-cyano-5-fluoro-benzoyl chloride of the formula (II) by elimination of water from 2,4-dichloro-5-fluoro-3-N-hydroxyiminomethyl-benzoic acid (III) with simultaneous conversion of the carboxylic acid function into the carbonyl chloride according to the following equation:

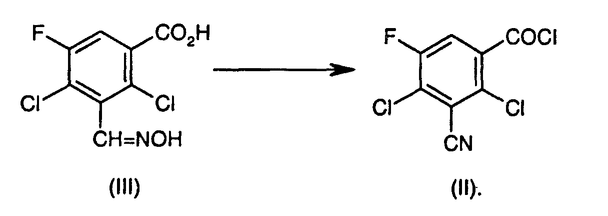

5. 2,4-Dichloro-5-fluoro-3-N-hydroxyimino-methylbenzoic acid of the formula (III)

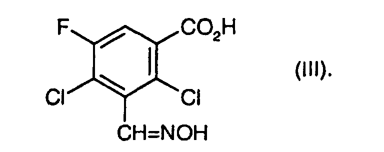

6. Process for the preparation of 2,4-dichloro-5-fluoro-3-N-hydroxyiminomethyl-benzoic acid of the formula (III) according to Claim 5, by reaction of 2,4-dichloro-5-fluoro-3-formyl-benzoic acid of the formula (IV) with hydroxylamine according to the following equation:

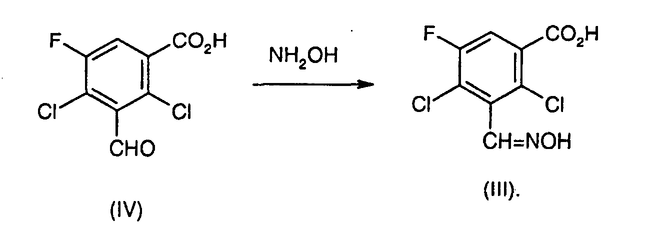

7. Process for the preparation of 2,4-dichloro-3-cyano-5-fluoro-benzoic acid (IX)
a) by elimination of water from 2,4-dichloro-5-fluoro-3-N-hydroxyiminomethyl-benzoic acid of the formula (III) according to the following equation 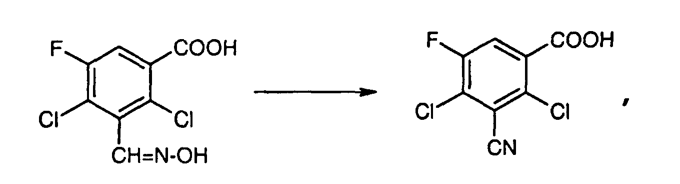
b) by reaction of 2,4-dichloro-5-fluoro-3-formylbenzoic acid of the formula (IV) with hydroxylamine in the presence of formic acid according to the following equation

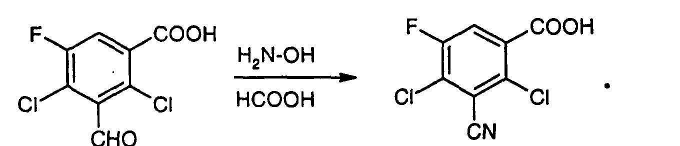

8. 2,4-Dichloro-5-fluoro-3-formyl-benzoic acid of the formula (IV)

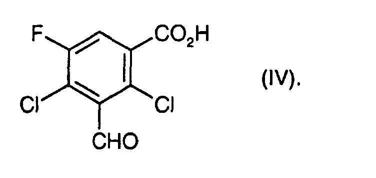

9. Process for the preparation of 2,4-dichloro-5-fluoro-3-formyl-benzoic acid of the formula (IV) according to Claim 7, 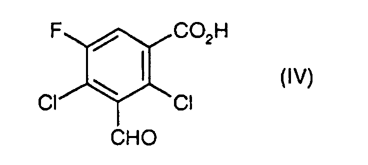
a) by hydrolysis of 2,4-dichloro-5-fluoro-3-dichloromethyl-benzoic acid or
b) by hydrolysis of 2,4-dichloro-5-fluoro-3-dichloromethyl-1-trichloromethylbenzene of the formula (VI),
in each case in the presence of acids and optionally in protic solvents.

10. 2,4-Dichloro-5-fluoro-3-dichloromethyl-1-trichloromethylbenzene of the formula (VI)

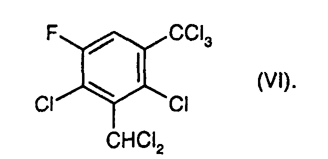

11. Process for the preparation of 2,4-dichloro-5-fluoro-3-dichloromethyl-l-trichloromethylbenzene of the formula (VI), 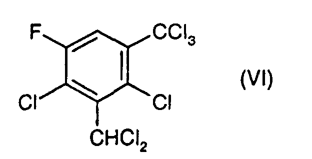 according to Claim 9 by side-chain chlorination of 2,4-dichloro-5-fluoro-1,3-dimethylbenzene (VII) to give 2,4-dichloro-5-fluoro-3-dichloromethyl-1-trichloromethylbenzene (VI) according to the following equation: 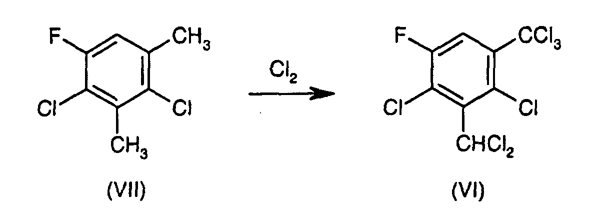 **characterized in that** the chlorination is carried out under free-radical conditions and/or at elevated temperature.

12. 2,4-Dichloro-5-fluoro-1,3-dimethylbenzene of the formula (VII)

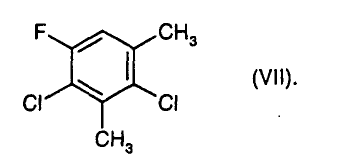

13. Process for the preparation of 2,4-dichloro-5-fluoro-1,3-dimethylbenzene of the formula (VII) according to Claim 11 by ring chlorination of 5-fluoro-1,3-xylene (VIII) to give 2,4-dichloro-5-fluoro-1,3-dimethylbenzene (VII) using chlorine gas, optionally in a diluent and in the presence of a catalyst according to the following equation:

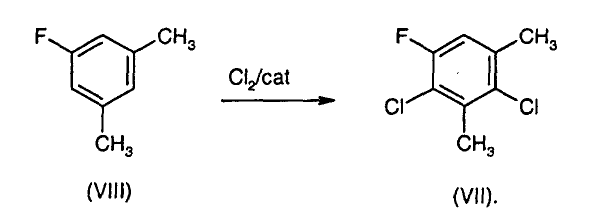

## Revendications

1. Fluorure de 3-cyano-2,4,5-trifluorobenzoyle de formule (I)

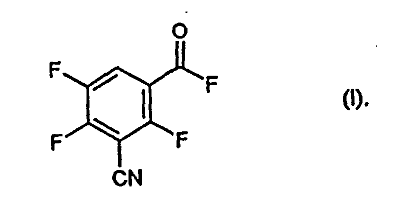

2. Procédé de production du fluorure de 3-cyano-2,4,5-trifluorobenzoyle de formule (I) suivant la revendication 1, **caractérisé en ce qu'**on conduit la réaction nucléophile du chlorure de 3-cyano-2,4-dichloro-5-fluorobenzoyle avec un agent de fluoruration conformément au schéma suivant :

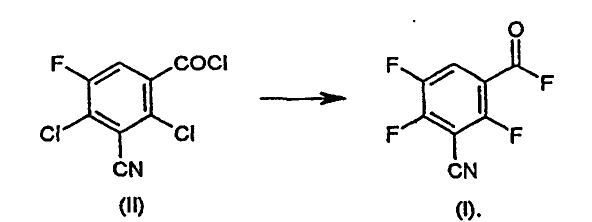

3. Utilisation du fluorure de 3-cyano-2,4,5-trifluorobenzoyle pour la production du chlorure de 3-cyano-2,4,5-trifluorobenzoyle par réaction du fluorure de 3-cyano-2,4,5-trifluorobenzoyle avec des agents de chloration, éventuellement en présence de diluants.

4. Procédé de production du chlorure de 2,4-dichloro-3-cyano-5-fluorobenzoyle de formule (II) par élimination d'eau de l'acide 2,4-dichloro-5-fluoro-3-N-hydroxyimino-méthylbenzoïque (III) accompagnée de la transformation de la fonction acide carboxylique en chlorure d'acide carboxylique d'après le schéma suivant :

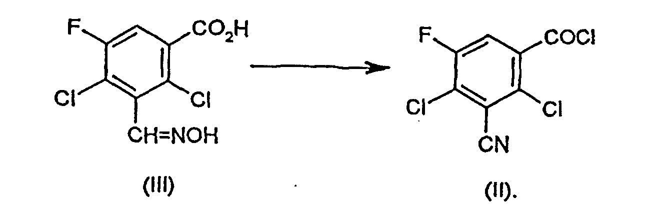

5. Acide 2,4-dichloro-5-fluoro-3-N-hydroxyiminométhylbenzoique de formule (III)

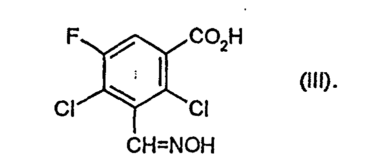

6. Procédé de production de l'acide 2,4-dichloro-5-fluoro-3-N-hydroxyiminométhylbenzoïque de formule (III) suivant la revendication 5, par réaction de l'acide 2,4-dichloro-5-fluoro-3-formylbenzoique de formule (IV) avec l'hydroxylamine d'après le schéma suivant :

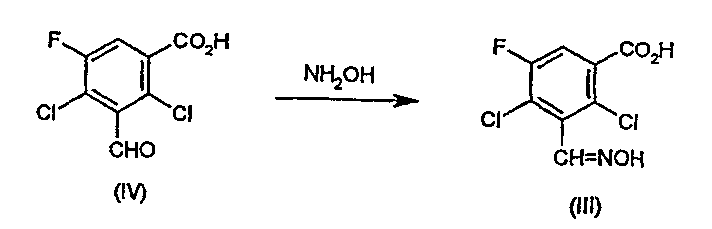

7. Procédé de production de l'acide 2,4-dichloro-3-cyano-5-fluorobenzoïque (IX)
a) par élimination d'eau de l'acide 2,4-dichloro-5-fluoro-3-N-hydroxyiminométhylbenzoïque de formule (III) d'après le schéma suivant : 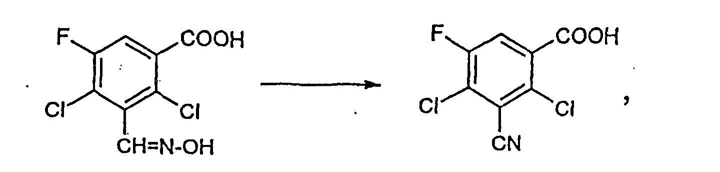
b) par réaction de l'acide 2,4-dichloro-5-fluoro-3-formylbenzoïque de formule (IV) avec l'hydroxylamine en présence d'acide formique d'après le schéma suivant :

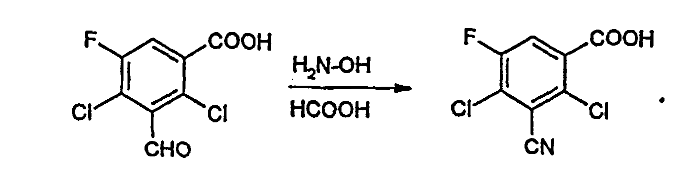

8. Acide 2,4-dichloro-5-fluoro-3-formylbenzoïque de formule (IV)

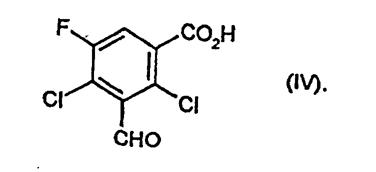

9. Procédé de production de l'acide 2,4-dichloro-5-fluoro-3-formylbenzoïque de formule (IV) suivant la revendication 7, 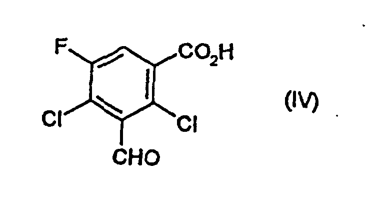
a) par saponification de l'acide 2,4-dichloro-5-fluoro-3-dichlorométhylbenzoïque
ou
b) par saponification du 2,4-dichloro-5-fluoro-3-dichlorométhyl-1-trichlorométhylbenzène de formule (VI),
dans chaque cas en présence d'acides et, le cas échéant, dans des solvants protiques.

10. 2,4-dichloro-5-fluoro-3-dichlorométhyl-1-trichlorométhylbenzène de formule (VI)

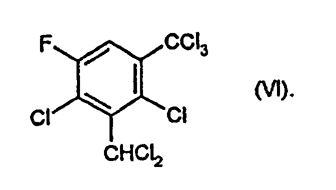

11. Procédé de production du 2,4-dichloro-5-fluoro-3-dichlorométhyl-1-trichlorométhylbenzène de formule (VI), 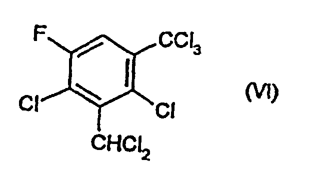 suivant la revendication 9 par chloration en chaîne latérale du 2,4-dichloro-5-fluoro-1,3-diméthylbenzène (VII) pour former le 2,4-dichloro-5-fluoro-3-dichlorométhyl-1-trichlorométhylbenzène (VI) conformément au schéma suivant : 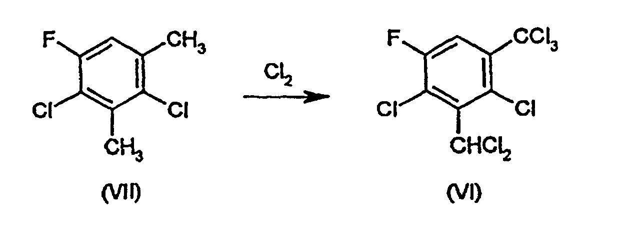 **caractérisé en ce qu'**on conduit la chloration dans des conditions radicalaires et/ou à température élevée.

12. 2,4-dichloro-5-fluoro-1,3-diméthylbenzène de formule (VII)

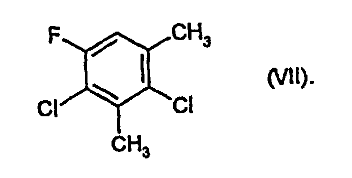

13. Procédé de production du 2,4-dichloro-5-fluoro-1,3-diméthylbenzène de formule (VII) suivant la revendication 11, par chloration du noyau du 5-fluoro-1,3-xylène (VIII) en 2,4-dichloro-5-fluoro-1,3-diméthylbenzène (VII) avec du chlore gazeux, le cas échéant dans un diluant et en présence d'un catalyseur, conformément au schéma suivant :
